# EUROPEAN PATENT APPLICATION

(11) **EP 1 913 882 A1**
(43) Date of publication of application: **23.04.2008**
(21) Application number: 07105004.1
(22) Date of filing: 27.03.2007
(51) Int. Cl.: A61B 17/22, A61M 25/10

(54) **Flexible and rigid catherer resector balloon**

(30) Priority: 16.10.2006 TR 200605770; 14.12.2006 US 610941
(71) Applicant: Y. K. K. Saglik Hizmetleri Ltd. Sti., 34365 Istanbul (TR)
(72) Inventor: Karakoca, Yalcin Y. K. K. SAGLIK HIZMETLERI LTD. STI., 34365, Istanbul (TR)
(74) Representative: Iskender, Ibrahim

(57) **Abstract**

The present invention relates to resector balloons (1) employed in treating endoluminal-endobronchial tumoral lesions and endovascular occlusions encountered in blood vessels and in other hollow tube-like organs (7), such as trachea, windpipe, food pipe, urinary tract, bile ducts. Said resector balloon (1) is composed of a resection tip (2); a resection part (3) that is swollen or inflated in such tube-like organs (7) and is displaced or moved back and forth therein to provide tumor resection; a hardening surface (4) provided on the outer surface of said resection part (3) to shave and destroy such tumoral tissues; a catheter section (5) providing access to an endoluminal site; and an injection terminal (6) capable to inflate said resection part (3) by injecting air or fluid.

## Description

### Technical Field

The present invention relates to resector balloons used in treating endoluminal-endobronchial tumoral lesions and endovascular occlusions, encountered in blood vessels and in other hollow tube-like organs, such as trachea, windpipe, food pipe, urinary tract, bile ducts.

The present invention more particularly relates to flexible and rigid resector balloons, which comprise a tip section with a variable diameter and length; a balloon section capable of tissue shaving and resectioning; a catheter section providing access to an endoluminal site, through a bronchoscope or any other endoscope; and an injector terminal to be equipped with an injector, used to inflate such balloon by means of injecting air or fluid thereto.

### Background of Invention

Disorders known as endoluminal tumoral lesions and endovascular occlusions are encountered in blood vessels and in other hollow tube-like organs, such as trachea, windpipe, food pipe, urinary tract, bile ducts. Such organs, except the bile duct, have the common feature of accessing directly to the exterior. Whilst being different from other organ systems, the blood vessel system incorporates the entire vascular system with primarily the coronary vessels within the human body. There are internal or external (i.e. internally or externally applied) treatment methods for tumoral lesions, contracting the lumen and growing inwardly so as to form barriers in the respiratory tract, food pipe and urinary tract.

Such disorders causing to the narrowing and occlusion of the trachea are treated in chest diseases and thoracic surgery departments. Another significant point in such diseases leading to suffocation of patients are infections, developing posterior to such disorders and insistent unless the occlusion is treated. In cases where no narrowness is observed on such suffocations, the factor causing to death in patients is the infectious state behind the narrowness. And any bleeding to occur on the tumor that blocks the trachea and any dependent respiratory insufficiency are the death factors established in patients with lung tumors. More correctly, the cause of death in 65 to 70% of lung cancer patients is the complications caused by the tumors occluding the trachea.

Such kind of occlusions caused by lung cancer and benign tumors emerge during the diagnosis stage or the progressing phases of disease. 15% of newly-diagnosed lung cancer patients are determined at the surgical treatment limit, in other words, only 15% of lung cancer patients are diagnosed timely to allow their surgical treatment; but the remaining 85% lose this surgical treatment opportunity during the diagnosis stage. These patients become incurred to treatment methods, which are actually more severe than surgical approaches. Besides surgical and oncological treatments, there are multimodal treatments available, including therapeutic bronchoscopy approaches for instance, providing intrabronchial passage (i.e. maintaining the bronchus interior in an non-occluded state) and eliminating external tumor pressure towards the bronchus interiors.

It is a known fact now that oncological therapy methods such as chemotherapy and radiotherapy have quite limited benefits on the lung cancer therapy and that the 5-year survival rates of patients have increased from 8% to 14% in the last twenty-five years. This 6% increase is attributed to early diagnosis techniques developed for lung cancer, rather than such oncological therapy methods. The therapeutic bronchoscopy methods employed in treating endobronchial tumors, which determine the survival rates of lung cancer patients, deteriorate their life quality, and at the same time, makes up almost 65 to 70% of death causes must be used more efficiently under the multimodal treatment principles.

Therapeutic bronchoscopy methods used in the therapy of endobronchial tumors include laser, cautery, argon, cryo, stent, and balloon treatments. Such treatment methods applied in accompany with rigid bronchoscopy are essentially based on tumor resection, i.e. on mechanically sectioning and removal of the tumor; coagulation, i.e. reducing the size of tumor by means of heat energy supplied by the laser and cautery; cryo, i.e. freezing, then mechanically sectioning and removal of the tumor.

In balloon applications among such endobronchial treatment methods, the balloon is used as a tampon to dilate a narrowed bronchus and to stop bleeding of a bleeding site therein. Balloon dilatation is used to dilate narrowed bronchi, and particularly to dilate the bronchus lumen before a stent is placed against the pressure of a tumor exerted from the exterior into the bronchus. And for controlling any hemorrhage due to a tumor tissue presence in the trachea, balloon tamponades are applied. Beside the trachea applications, balloons are also applied to dilate any narrowness in the esophagus and ureters, and to control any hemorrhage therein.

Laser-, cautery-, and cryo-based devices are also used in treating endoluminal lesions, that is to say, in treating any occlusive tumors particularly present in the trachea, and also in the esophagus and ureters.

In the trachea, for instance, the mechanical resection of an endoluminal tumor may be carried out by means of rigid bronchoscopy, and it is also possible to perform other laser- or cautery-based techniques. There are, however, some difficulties in applying laser-, cautery-, or cryo-based techniques in resectioning endobronchial lesions that cause narrowness in the two main bronchi entrance of the tracheal carina, due to the anatomy of the bronchus entrance. Therefore, during a therapeutic bronchoscopy application, it is often deemed adequate to provide a small-diameter opening in the lumen, resulting in an incomplete endobronchial resectioning. And in other circumstances, some stent applications are carried out, which do not conform with the anatomy of the tracheal carina.

Additionally, such aforementioned laser- or cryo-based therapeutic bronchoscopy methods are somewhat risky, in that they bear the potential of occluding and narrowing the interior of hollow tube-like organs such as trachea, bronchi, esophagus, etc. On this account, these methods are not efficient enough in some cases.

The most substantial limiting factors of endobronchial treatment methods used solely in the most developed cancer treatment centers worldwide are the factors related to the localization of tumor. Whilst it is relatively simple to apply laser-, cautery-, or cry-based resection methods in relatively large airways such as the trachea; in resectioning endoluminal tumors occluding the upper lobe, medial lobe, lingula, lower lobe, and their segments in more distal airways, complication risks associated with laser-, cautery-, argon-, cryo-based and mechanical resection applications increase and no complete endoluminal resection is achieved.

Particularly in rigid bronchoscopy applied under general anesthesia without suppressing the patient's respiration, the endoluminal lesions cannot be reached to in the lobe and segment bronchi, and no standard treatment approaches can be determined.

The use of balloons for resection purposes, besides their dilatation- and tampon-oriented use in treating endobronchial tumoral lesions, and thus the resector balloon concept is not available yet in the medicine literature.

Apart from endoluminal lesions, the treatment of endovascular occlusions is one of the most significant problems of medicine. Especially the balloon angioplasty and metal stent placement among the treatments applied to coronary heart diseases are quite widespread, but substantially expensive. Medicated or unmedicated stents applied to eliminate any occlusion in coronary vessels are being widely used.

Accordingly, easily-applied and low-cost methods are required for use in treating specifically the coronary artery diseases with the purpose of eliminating any occlusion in coronary vessels by ensuring the dilatation of the latter.

### Brief Description of Invention

Under the light of the foregoing statements, the objective of the present invention is to develop a resector balloon employed in treating endoluminal-endobronchial tumoral lesions and endovascular occlusions, encountered in blood vessels and in other hollow tube-like organs, such as trachea, windpipe, food pipe, urinary tract, bile ducts.

Another objective of the present invention is to develop an alternative tumor resection method that eliminates the risks of laser- or cryo-based therapeutic bronchoscopy methods, which bear the potential of occluding and narrowing the interior of hollow tube-like organs such as trachea, bronchi, windpipe, food pipe.

A further objective of the present invention is to provide the complete removal of tumor(s) from both main bronchi entrances of the tracheal carina in conformance with the bronchus anatomy.

Another objective of the present invention is to provide a bronchus with its normal cross-sectional width and to ensure the widest stent placement possible, when stent indication is present.

Yet another objective of the present invention is to reduce the complication risks associated with resectioning of endoluminal tumors occluding the upper lobe, medial lobe, lingula, lower lobe, and their segments in distal airways.

Yet a further objective of the present invention is to provide access to endoluminal lesions present in such lobes and segmental bronchi under general anesthesia, and to allow for assigning or determining standard treatment approaches.

Still a further objective of the present invention is to develop a resector balloon for use in treating endovascular occlusions and particularly the coronary artery disease, by providing dilatation in coronary vessels, and thereby eliminating any occlusions in the latter.

Still another objective of the present invention is to develop a mechanical tumor resection method, which is conveniently-applicable with respect both to the cardiology specialist and the affected patient, and which is also low-cost and easily-affordable.

In order to achieve the aforesaid objectives, a resector balloon is developed comprising a resection tip with variable diameter and length; a resection part capable of tissue shaving and resectioning; a catheter section providing access to an endoluminal site, through a bronchoscope or any other endoscope; and an injection terminal to be equipped with an injector, used to inflate said resection part by means of injecting air or fluid thereto.

### Brief Description of Figures

Figure 1 illustrates the present device within a tube-like organ prior to swelling or inflation.
Figure 2 illustrates the present device within a tube-like organ after becoming swollen or inflated.

### Reference Numbers

**1.** Resector balloon
**2.** Resection tip
**3.** Resection part
**4.** Hardening surface
**5.** Catheter section
**6.** Injection terminal
**7.** Tube-like organ.

### Detailed Description of Invention

The present invention relates to flexible and rigid resector balloons (1) developed for use in treating endoluminal-endobronchial tumoral lesions and endovascular occlusions, encountered in blood vessels and in other hollow tube-like organs (7), such as trachea, windpipe, food pipe, urinary tract, bile ducts.

Said resector balloon (1) is composed of a resection tip (2) with variable diameter and length; a resection part (3) capable of tissue shaving and resectioning; a catheter section (5) providing access to an endoluminal site, through a bronchoscope or any other endoscope; and an injection terminal (6) to be equipped with an injector, used to inflate said resection part (3) by means of injecting air or fluid thereto.

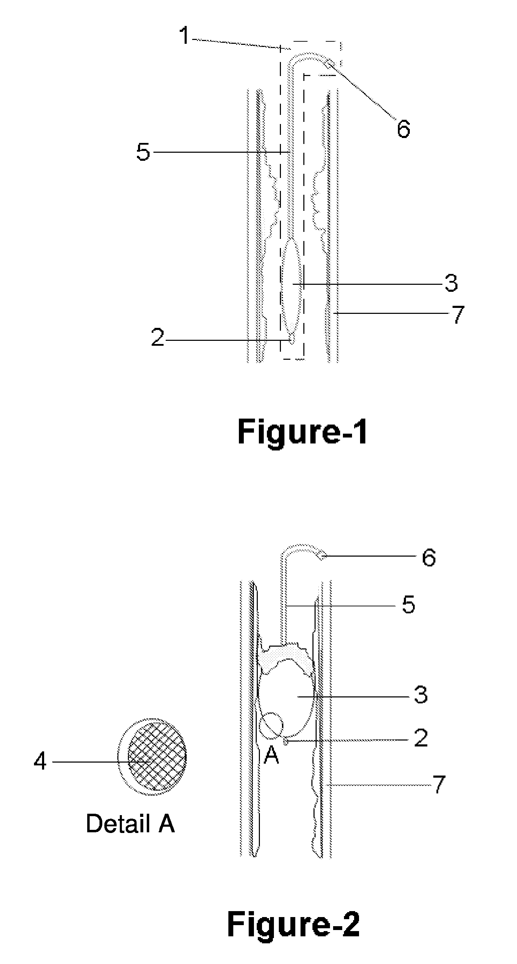

Figure 1 illustrates the present resector balloon (1) within a tube-like organ (7) before it is swollen or inflated. In the present resector balloon (1), said resection part (3) is swollen or inflated after being placed into hollow tube-like organs (7), such as trachea, bronchi lumen, windpipe, food pipe, urinary tract, bile ducts against endoluminal lesions accompanied by exterior pressure.

Figure 2 illustrates the present resector balloon (1) within a tube-like organ (7) after it is swollen or inflated. The resection part (3) of the resector balloon (1) is swollen or inflated in such tube-like organs (7) so as to provide them with dilatation, and once a tumor is passed or crossed, the tumor resection process is carried out by means of moving the present device back and forth.

Since a hardening surface (4) provided on the outer surface of said resection part (3) is capable to perform resectioning thanks to its special coating, it becomes possible to shave and destroy a tumor tissue and hence to treat any endoluminal-endobronchial tumoral lesions and endovascular occlusions.

Such tumor tissues are removed out in/with the resector balloon (1). Said process is repeated until such tube-like organ (7) is completely opened and cleaned from the tumoral formation. Any hemorrhage to occur during this process can be stopped by means of the tamponning affect of the present resector balloon (1). This endoluminal resector balloon (1) approach is a treatment method applicable when tube-like organs (7) are occluded by tumors.

The endoluminal-endobronchial resector balloon (1) may be applied both through a flexible bronchoscope and a rigid bronchoscope, with the latter case being relatively safer. Alternatively, any flexible resector balloon (1) accompanied by a rigid application tube can be used likewise and may be considered in the same device class. By rotating a rigid resector balloon (1) to the right and left, in addition to displacing it back and forth, it becomes possible to perform resectioning processes in endoluminal, endobronchial, endotracheal, and endoesophageal tumoral lesions.

It is further possible to completely clean from tumors both main bronchi entrances of the tracheal carina by sticking to the anatomy (of bronchi) thanks to the present resector balloon (1). As a result, the bronchus is restored to its normal cross-sectional width and the widest stent placement is ensured, when stent indication is mentionable.

In addition, the present resector balloon (1) reduces the complication risk in resectioning endoluminal tumors occluding the upper lobe, medial lobe, lingula, lower lobe, and their segments in distal airways, and thus is applied as a treatment method that is more efficient than all other techniques.

Thanks to the present resector balloon (1), the endoluminal lesions present in such lobes and segmental bronchi are accessed particularly under general anesthesia and standard treatment approaches are assigned accordingly.

Apart from endoluminal lesions, the use of the endovascular resector balloon (1) in treating endovascular occlusions allows for new horizons in treating coronary artery diseases, as an alternative method to dilatation and stent placement procedures. Said resector balloon (1) eliminates any occlusions in coronary vessels by providing dilatation to the latter.

Both the simple application, and the low-cost and easily-affordable features of the resector balloon (1) make it convenient not only for the cardiology specialists, but also for affected patients.

The protection scope of this Application is set forth in the following claims and is not to be restricted with the disclosures given above for illustrative purposes only. It is obvious that a person skilled in the relevant art can produce the currently-disclosed novelty by making use of similar embodiments and/or can apply the subject embodiment to other fields with similar purposes. Therefore it is also clear that such embodiments shall lack the novelty and inventive step criterion.

## Claims

1. A flexible and rigid resector balloon (1) for use in treating endoluminal-endobronchial tumoral lesions and endovascular occlusions encountered in blood vessels and in other hollow tube-like organs (7) such as trachea, windpipe, food pipe, urinary tract, bile ducts, said resector balloon (1) being **characterized in** comprising
- a resection part (3), which is inflated in such tube-like organs (7) so as to provide dilatation therein (7), and once a tumor is passed or crossed, performs the tumor resection process by means being displaced or moved back and forth;
- a catheter section (5), which provides access to an endoluminal site through a bronchoscope or any other endoscope; and
- an injection terminal (6) that may be equipped with an injector, which is used to inflate said resection part (3) by injecting air or fluid.

2. A flexible and rigid resector balloon (1) according to Claim 1, **characterized in** further comprising a hardening surface (4), which is provided on the outer surface of said resection part (3), and which destroys by shaving any tumoral tissue within said tube-like organs (7).

3. A flexible and rigid resector balloon (1) according to Claim 1, **characterized in** further comprising a resection tip (2), which is provided on the tip of said resection part (3) and is in connection with the resection part (3).
